# EUROPEAN PATENT SPECIFICATION

(11) **EP 4 471 422 B1**
(45) Date of publication and mention of the grant of the patent: **01.07.2026**
(21) Application number: 23900110.0
(22) Date of filing: 21.12.2023
(51) Int. Cl.: G01N 30/88, G01N 30/96, G01N 21/25, G01N 33/14, G01N 33/00, G01N 30/86, G01N 15/0205

(54) **RAPID SCREENING AND EVALUATION METHOD SUITABLE FOR RAW MATERIAL OF SEAWEED NEAR-WATER BEVERAGE**
SCHNELLES SCREENING- UND BEWERTUNGSVERFAHREN FÜR ROHMATERIAL EINES SEETANG-NAHWASSERGETRÄNKS
PROCÉDÉ DE CRIBLAGE ET D'ÉVALUATION RAPIDE APPROPRIÉ POUR UNE MATIÈRE PREMIÈRE DE BOISSON PROCHE DE L'EAU À BASE D'ALGUES

(30) Priority: 29.12.2022 CN 202211708919
(43) Date of publication of application: 04.12.2024
(73) Proprietor: Tsingtao Brewery Co., Ltd., Shibei District Qingdao, Shandong 266000 (CN)
(72) Inventor: YIN, Hua, Qingdao, Shandong 266000 (CN); HUANG, Kexing, Qingdao, Shandong 266000 (CN); HUANG, Shuli, Qingdao, Shandong 266000 (CN); HU, Shumin, Qingdao, Shandong 266000 (CN); QIAN, Zhonghua, Qingdao, Shandong 266000 (CN); CHANG, Zongming, Qingdao, Shandong 266000 (CN); CHEN, Lu, Qingdao, Shandong 266000 (CN); LIU, Jia, Qingdao, Shandong 266000 (CN); YANG, Zhaoxia, Qingdao, Shandong 266000 (CN); XING, Lei, Qingdao, Shandong 266000 (CN); WANG, Chenghong, Qingdao, Shandong 266000 (CN); ZHANG, Cui, Qingdao, Shandong 266000 (CN)
(74) Representative: J A Kemp LLP
(86) International application number: PCT/CN2023/140571
(87) International publication number: WO 2024/120542

(56) References cited:
- CN-A- 106 472 686
- CN-A- 110 850 049
- CN-A- 111 109 409
- CN-A- 111 272 895
- CN-A- 115 932 190
- JP-A- 2009 247 283
- JP-A- 2015 226 533
- US-A1- 2005 217 596
- US-A1- 2008 124 429
- US-A1- 2008 124 429

## Description

The present application claims the priority benefit of Chinese application No. 202211708919.7, filed on December 29, 2022, entitled "Rapid Screening and Evaluation Method for Raw Material of Seaweed Near Water Beverage".

### TECHNICAL FIELD

The present application belongs to the food technology field, and particularly relates to a rapid screening and evaluation method for raw material of seaweed near water beverage.

### BACKGROUND ART

A "near water" beverage is a product that is between drinking water, fruit juice, and functional drinks, and has a clear and transparent appearance and a certain functional characteristic. Seaweed is one of the oldest plants with the highest biomass in the world, and the primary bioactive substances are seaweed polysaccharides such as agar, carrageen, algin and the like, which are important ingredients of seaweed cell walls, but have the characteristics of high viscosity, strong gel property and the like, affecting its application effect.

Due to the poor solubility, low active ingredient conversion, and easy to have a fishy smell of seaweed enzyme powder, currently, the seaweed food is mainly used in products such as seaweed noodles, seaweed cans, and seaweed tablets etc., and cannot be used in near water beverages effectively. The near water beverage is required to be transparent in appearance and free of odor. Therefore, seaweed near water beverage has a higher requirement on quality such as solubility, active ingredients, and sense of the seaweed enzyme powder.

Traditional evaluation methods for seaweed enzyme powder primarily perform qualitative evaluation on appearance, color and sense, lacking solubility and functional ingredients evaluation. Furthermore, the quality of the raw material is typically evaluated based on the final beverage, with hysteresis. Additionally, traditional methods focus on evaluating the raw material, concentrated solutions prepared by the raw material, or the beverage itself, without considering how the quality of the raw material affects the shelf life of the beverage, which often results in products that appear fine when leaving the factory but may develop issues such as cloudiness or a strong fishy odor during shelf life. Therefore, developing evaluation and screening techniques for seaweed enzyme powder that are suitable for near-water beverages is particularly important.

US20080124429A1 discloses a nutritious, tasty and affordable drink made from the sap of Kappaphycus alvarezii seaweed which is readily cultivable. The drink resembles coconut water in appearance and taste and is rich in potassium. It also contains an adequate proportion of the daily requirement of iodine besides many other useful minerals such as magnesium, calcium, sodium, zinc, phosphorous and iron while having low concentrations of toxic elements such as lead and chromium. The process of refining sap, which enables the seaweed drink to be palatable and widely acceptable, besides bestowing adequate shelf life, is also described. The process of preparation is integrated with preparation of carrageenan and plant nutrient thereby making optimum use of the seaweed and minimizing waste.

Reference may also be made to US 2005/0217596 A1, which discloses a marine algae powder for food and drink in which tea leaf powder is mixed with Porphyra marine algae powder and/or Undaria marine algae powder so that the smell of the sea is abated while nutritional ingredients and health functional ingredients are preserved.

### SUMMARY OF THE INVENTION

The present application provides a rapid screening and evaluation method for raw material of seaweed near water beverage, which can solve the technical problem that the traditional evaluation technology for seaweed enzyme powder is only qualitative elevation and cannot guarantee the requirement of solubility, sensory and functional ingredients of the raw materials of the seaweed near-water beverage.

According to the present invention, there is provided with a rapid screening and evaluation method for raw material of seaweed near water beverage as defined in the independent claim.

In an example, the step of analyzing the appearance, the sense, and the particle size of the seaweed enzyme powder as raw material includes:
the appearance: when the color is light yellow to light brown, the seaweed enzyme powder is marked as grade I; when the color is brown, the seaweed enzyme powder is marked as grade II; and when the color is dark brown, the seaweed enzyme powder is marked as grade III;
the sense: when the smell is slight fishy smell to mild fishy smell, the seaweed enzyme powder is marked as grade I; when the smell is moderate fishy smell, the seaweed enzyme powder is marked as grade II; and when the smell is strong fishy smell, the seaweed enzyme powder is marked as grade III; and
the particle size: when particle size≤50µm, the solubility is excellent; when 50µm<particle size<80µm, the solubility is good; when 80µm≤particle size<120µm, the solubility is average; and when particle size≥120µm, the solubility is poor.

In an example, the step of analyzing the appearance, the sense, and the percentage with molecular weight less than 10 thousand of the 3% seaweed stock solution obtained based on the seaweed enzyme powder includes:
the color (appearance): when the color is light yellow, the stock solution is marked as grade I; when the color is brown, the stock solution is marked as grade II; and when the color is dark brown, the stock solution is marked as grade III;
the sense: when the smell is slight fishy smell, the stock solution is marked as grade I; when the smell is mild fishy smell, the stock solution is marked as grade II; and when the smell is strong fishy smell, the stock solution is marked as grade III; and,
the percentage with molecular weight less than 10 thousand: when percentage<50%, the solubility is poor; when 50%≤percentage≤80%, the solubility is average; and when percentage>80%, the solubility is good.

In an example, the step of analyzing the appearance, the sense, the turbidity, and the functional ingredients of the first seaweed near water beverage obtained based on the 3% seaweed stock solution includes:
the color (appearance): when the color is colorless, the first seaweed near water beverage is marked as grade I; and when the color is light yellow, the first seaweed near water beverage is marked as grade II;
the sense: when the smell is free of fishy smell, the first seaweed near water beverage is marked as grade I; when the smell is slight fishy smell, the first seaweed near water beverage is marked as grade II; and when the smell is strong fishy smell, the first seaweed near water beverage is marked as grade III; and
the turbidity: when turbidity<0.2, the first seaweed near water beverage is marked as grade I; when 0.2≤turbidity≤0.5, the first seaweed near water beverage is marked as grade II; and when turbidity>0.5, the first seaweed near water beverage is marked as grade **III.**

In an example, the functional ingredients include a measured light absorption value OD235 at 235nm of the first seaweed near water beverage and a content of oligosaccharide contained in the first seaweed near water beverage.
when light absorption value OD235>0.20, the first seaweed near water beverage is marked as grade I; when 0.15≤light absorption value OD235≤0.20, the first seaweed near water beverage is marked as grade II; and when light absorption value OD235<0.15, returning the raw material to a raw material plant directly; and,
when content of oligosaccharide>45ppm, the first seaweed near water beverage is marked as grade I; when 25ppm≤content of oligosaccharide≤45ppm, the first seaweed near water beverage is marked as grade II; and when content of oligosaccharide<25ppm, returning the raw material to the raw material plant directly.

In an example, the enhanced seaweed near water beverage is a beverage obtained by exposing the first seaweed near water beverage to sunlight for 4 hours; wherein, the first seaweed near water beverage is a 0.01% seaweed enzyme powder solution obtained by diluting the 3% seaweed stock solution.

In an example, the step of analyzing the appearance, the sense, the turbidity, and the functional ingredients of the enhanced seaweed near water beverage includes:
the color (appearance): when the color is colorless and transparent, the enhanced seaweed near water beverage is marked as grade I; when the color is colorless with slight floccules, the enhanced seaweed near water beverage is marked as grade II; and when the color is light yellow with slight floccules, the enhanced seaweed near water beverage is marked as grade III;
the sense: when the smell is free of fishy smell, the enhanced seaweed near water beverage is marked as grade I; when the smell is slight fishy smell, the enhanced seaweed near water beverage is marked as grade II; and when the smell is strong fishy smell, the enhanced seaweed near water beverage is marked as grade III; and
the turbidity: when turbidity<0.2, the enhanced seaweed near water beverage is marked as grade I; when 0.2≤turbidity≤0.5, the enhanced seaweed near water beverage is marked as grade II; and when turbidity>0.5, the enhanced seaweed near water beverage is marked as grade **III.**

In an example, the functional ingredients include a measured light absorption value OD235 at 235nm of the enhanced seaweed near water beverage and a content of oligosaccharide contained in the enhanced seaweed near water beverage.

In an example, the functional ingredients specifically are:
when light absorption value OD235>0.20, the enhanced seaweed near water beverage is marked as grade I; when 0.15≤light absorption value OD235≤0.20, the enhanced seaweed near water beverage is marked as grade II; and when light absorption value OD235<0.15, returning the raw material to the raw material plant directly; and,
when content of oligosaccharide>45ppm, the enhanced seaweed near water beverage is marked as grade I; when 25ppm≤content of oligosaccharide≤45ppm, the enhanced seaweed near water beverage is marked as grade II; and when content of oligosaccharide<25ppm, returning the raw material to the raw material plant directly.

When the raw material of the near water beverage is screened and evaluated, the closer each parameter (for example, the appearance, the sense, the particle size, the molecular weight, the turbidity, the light absorption value, and the content of oligosaccharide) is to the grade I, the better the quality is, the more conducive to the production. Therefore, the grade I indexes are preferred.

Compared with the prior art, the present application has the following advantages and positive effects:
The rapid screening and evaluation method for raw material of the seaweed near water beverage provided by at least one example of the present application comprehensively evaluates the seaweed raw material of the near water beverage for the first time, including the appearance, sense, solubility, and functional ingredients, and grades according to the index parameters;
the rapid screening and evaluation method for raw material of the seaweed near water beverage provided by at least one example of the present application can rapidly and comprehensively determine the advantages and disadvantages of one kind of seaweed raw material, and formulate a next production process according to the advantages and disadvantages, so as to guarantee that the seaweed near water beverage is clear and transparent in appearance, is odor-free and has significant functions;
the rapid screening and evaluation method for raw material of the seaweed near water beverage provided by at least one example of the present application contains evaluation of the raw material, evaluation of an initial beverage, and evaluation of the beverage in the entire shelf life, and the evaluation phases are more comprehensive, thereby providing a technical base for ensuring the product quality comprehensively.

### DETAILED DESCRIPTION OF THE EMBODIMENTS

The technical solutions in the embodiments of the present application will be described clearly and completely as below. It is obvious that the described embodiments are only part of the embodiments of the present application, not all of the embodiments. Based on the embodiments in the present application, all other embodiments obtained by ordinary skill in the art without creative work shall fall within the scope of the claims.

The unit of molecular weight is Dalton, and the absorbance value has no unit, which are known to those skilled in the art. Concentrations in the present application refer to mass concentrations. For example, the "3% seaweed stock solution" means that the mass concentration of the seaweed enzyme powder in the seaweed stock solution is 3%, and the "0.01% seaweed enzyme powder solution" means that the mass concentration of the seaweed enzyme powder in the solution is 0.01%. Besides, the oligosaccharide in the present application refers to a compound formed by 3-10 monosaccharide molecules linked by glucosidic bonds.

The turbidity in the present application is EBR turbidity, which is usually used to determine the turbidity degree of beer, and is a measuring standard to describe the stability of beer. EBC is an abbreviation of "European Brewery Convention", which means the European Brewery Convention. The turbidity of the solution in the present application is also determined by using the EBC turbidity, and during an actual operation, the turbidity of the near water beverage may be measured by using a turbidity analyzer.

The appearance and color in the present application are measured by an EBC colorimeter. A test method includes: injecting a sample (solution) into a cuvette of the EBC colorimeter; comparing with a standard EBC color disc; and reading the chromaticity of the sample visually or automatic digital display, with represented by the chromaticity unit EBC. Wherein, colorless means chromaticity <2 EBC; light yellow or light brown means 2EBC≤chromaticity<15EBC; brown means 15EBC≤chromaticity≤25EBC; and dark brown means chromaticity >25 EBC.

### Example 1 Evaluation of seaweed enzyme powder

### 1.1 Particle size analysis of the seaweed enzyme powder

Particle size analysis method: A Malvern Mastersizer 3000 laser diffraction particle size analyzer was used for analysis. First, ensure that a host machine and accessories connected at the lower right corner of the software were selected normally. If multiple accessories were connected at the same time, select Aero S accessory. A dry dispersion system needed to be connected to a compressed gas and a vacuum system (vacuum cleaner). Before the dry test, it was needed to confirm that the compressed gas and the vacuum cleaner were both turned on to avoid insufficient gas pressure and vacuum failure. The maximum gas flow pressure in the software was 4 bar, and therefore, it was usually required that the pressure at a gas source outlet reaches 6 bar. Add 5 g of seaweed enzyme powder, select the "sampling injection" system to analyze, and evaluate the solubility of the seaweed enzyme powder according to the particle size range in table 1.

**Table 1**

| Particle size (Dx90) | ≤50 µm | 50 µm-80 µm | 80 µm-120 µm | ≥120 µm |
|---|---|---|---|---|
| Solubility evaluation | Excellent solubility | Good solubility | Ordinary solubility | Poor solubility |

### 1.2 Appearance and sensory evaluation of the seaweed enzyme powder

Appearance and sensory evaluation method: take an appropriate amount of seaweed enzyme powder sample and place in a clean and dry white ceramic plate, observe the color and luster thereof under natural light, and taste the seaweed enzyme powder to judge whether the seaweed enzyme powder had any fishy smell. Corresponding grades based on color and luster and fishy smell are given in the following table 2.

Since the seaweed enzyme powder was powder, the color was judged by naked-eye observation. When the color was light yellow to light brown, it was marked as grade I; when the color was brown, it was marked as grade II; and when the color was dark brown, it was marked as grade III. As the color in the example was observed by naked eyes, there was a slight subjective deviation; however, since the powder will be dissolved and diluted subsequently, the color judgment here only serves as an initial reference, allowing the deviation. For example, when there are 5 judges and 3 of them believe that the seaweed enzyme powder was light brown, it can be marked as grade I.

For sensory evaluation, a first reference with slight fishy smell was selected, and a second reference with strong fishy smell was selected; and each seaweed enzyme powder sample was respectively compared with the first reference and the second reference to determine its grade. When the smell was weaker than that of the first reference, it was judged as slight fishy smell to mild fishy smell, and marked as grade I; when the smell was between that of the first reference and the second reference, it was judged as moderate fishy smell, and marked as grade II; and when the smell was stronger than that of the second reference, it was judged as strong fishy smell, and marked as grade III. Similarly, since the powder will be dissolved and diluted subsequently, the sensory judgment for the powder here only serves as an initial reference, allowing a deviation. Therefore, the first and second reference and the judgment criteria were selected as long as they can meet the mass production requirements.

**Table 2**

| Appearance | Light yellow | Light brown | Brown | Dark brown |
|---|---|---|---|---|
| Grade judgment | Grade I | Grade I | Grade II | Grade III |
| Sense | Slight fishy smell | Mild fishy smell | Moderate fishy smell | Strong fishy smell |
| Grade judgment | Grade I | Grade I | Grade II | Grade III |

### Example 2 Evaluation of seaweed stock solution

Weigh 15 g of the seaweed enzyme powder and dissolve it with about 400 mL of water (pH about 7, and hardness ≤2 Germanic degrees); after completely dissolved, adjust the volume to 500 mL to prepare a 3% seaweed enzyme powder stock solution, i.e., obtain a 3% seaweed stock solution, where the mass concentration of the seaweed enzyme powder was 3%.

### 2.1 Appearance and sensory evaluation of the seaweed stock solution

An appearance and sensory evaluation method: place 100 mL of the seaweed stock solution in a clean and dry colorless transparent plastic cup; observe the color and luster under natural light and measure the color by an EBC colorimeter; and taste the seaweed stock solution to judge whether the seaweed stock solution had any fishy smell. Corresponding grades of color and fishy smell of the seaweed stock solution are shown in Table 3.

Wherein, for the color evaluation, inject the 3% seaweed stock solution into a cuvette of the EBC colorimeter, and compare it with a standard EBC color disc to read the chromaticity of the solution. When 2EBC≤chromaticity < 15EBC, it was judged as light yellow, and marked as grade I; when 15EBC≤chromaticity≤25EBC, it was judged that as brown, and marked as grade II; and when the chromaticity >25 EBC, it was judged as dark brown, and marked as grade III. It was worth understanding that when the chromaticity was less than 2 EBC, the seaweed stock solution was also marked as grade I.

For the sensory evaluation, a third reference with slight fishy smell and a fourth reference with strong fishy smell were set; and the two references were both selected in combination with the mass production process, and the mass concentrations of the seaweed enzyme powder thereof were both 3%. Wherein, the third reference was excellent in mass production test quality through a test, but the seaweed stock solution has slight fishy smell; and the fourth reference was unacceptable in mass production test quality through the test. The 3% seaweed stock solution in the example was respectively compared with the third reference and the fourth reference to determine its grade. When the smell was weaker than that of the third reference, it was judged as slight fishy smell, and marked as grade I; when the smell was stronger than that of the third reference but less than that of the fourth reference, it was judged as mild fishy smell, and marked as grade II; and when the smell was stronger than that of the fourth reference, it was judged as strong fishy smell, and marked as grade III.

In the present example and other examples, the sensory evaluation can be judged in a probabilistic manner. For example, 30 persons with normal taste senses were selected randomly to respectively judge the relations between the stock solution and the references; for example, when no fewer than 20 persons (2/3) believe that the smell was weaker than that of the third reference, it was judged as slight fishy smell, and marked as grade I; of course, other reliable methods can also be used for the judgment.

**Table 3**

| Appearance | Light yellow | Brown | Dark brown |
|---|---|---|---|
| Chromaticity, EBC | <15 | 15-25 | >25 |
| Grade judgment | Grade I | Grade II | Grade III |
| Sense | Slight fishy smell | Mild fishy smell | Strong fishy smell |
| Grade judgment | Grade I | Grade II | Grade III |

### 2.2 Analysis of the solubility of the seaweed stock solution

Analysis of molecular weight distribution of the seaweed enzyme powder: filter the 3% seaweed stock solution centrifugally by an ultrafiltration tube of 10 thousand molecular weight, and analyze the contents of the soluble solid of the liquid before and after the centrifugation.

An analysis method for the content of the soluble solid: drop the sample solution onto a disk of a refractometer, close the lid, analyze the sample, and record data.

Calculation of the proportion of the solid with a molecular weight less than 10 thousand: soluble solid of the below solution in the ultrafiltration tube/soluble solid of 3% seaweed stock solution*100. Table 4 provides the molecular weight less than 10 thousand proportion % of the 3% seaweed stock solution and corresponding grades of its solubility.

**Table 4**

| Molecular weight less than 10 thousand proportion | <50% | 50%-80% | >80% |
|---|---|---|---|
| Solubility judgment | Poor solubility | Ordinary solubility | Good solubility |

### Example 3 Analysis of the first seaweed near water beverage

The 3% seaweed stock solution was diluted to a 0.01% seaweed enzyme powder solution (it can be diluted with water in example 2) to obtain the first seaweed near water beverage.

### 3.1 Appearance and sensory evaluation of the first seaweed near water beverage

An appearance and sensory evaluation method: place 100 mL of the first seaweed near water beverage in a clean and dry colorless transparent plastic cup; observe the color and luster thereof under natural light and measure the color by an EBC colorimeter; and taste the flavor of the first seaweed near water beverage. Corresponding grades of color and luster and fishy smell of the first seaweed near water beverage are shown in Table 5.

Wherein, for the color evaluation, inject the first seaweed near water beverage into the cuvette of the EBC colorimeter, and compare with the standard EBC color disc to read the chromaticity of the solution. When chromaticity<2 EBC, it was judged as colorless, and marked as grade I; and when 2EBC≤chromaticity<15EBC, it was judged as light yellow, and marked as grade II.

For the sensory evaluation, carbonated purified water as a fifth reference and a six reference with strong fishy smell were set, wherein the six reference was a corresponding solution obtained by diluting the fourth reference with strong fishy smell in example 2 from 3% by mass concentration to 0.01% by mass concentration. The first seaweed near water beverage was respectively compared with the fifth reference and the sixth reference to determine its grade. When the smell was close to that of the fifth reference, it was judged as free of fishy smell, and marked as grade I; when the smell was between that of the fifth reference and that of the sixth reference, it was judged as slight fishy smell, and marked as grade II; and when the smell was stronger than that of the sixth reference, it was judged as strong fishy smell, and marked as grade **III.**

**Table 5**

| Appearance | Colorless | Light yellow | |
|---|---|---|---|
| Chromaticity, EBC | <2 | ≥2 | |
| Grade judgment | Grade I | Grade II | |
| Sense | Free of fishy smell | Slight fishy smell | Strong fishy smell |
| Grade judgment | Grade I | Grade II | Grade III |

### 3.2 Turbidity analysis

The turbidity of the first seaweed near water beverage was analyzed by using a turbidity analyzer, and its turbidity and corresponding range are shown in Table 6.

**Table 6**

| Turbidity of the seaweed product | <0.2 | 0.2-0.5 | >0.5 |
|---|---|---|---|
| Grade judgment | Grade I | Grade II | Grade III |

### 3.3 Evaluation of functional ingredients

An OD235 analysis method: take the first seaweed near water beverage, and measure the absorbance value of the first seaweed near water beverage at 235 nm, with distilled water as a reference.

An oligosaccharide content analysis method: oligosaccharide in the seaweed raw material was analyzed by using an ion chromatography (IC). Disaccharide to hexaose of mannuronic acid was used as a glycan standard sample for qualitative and quantitative analysis. The seaweed enzyme powder was prepared into a 0.01% solution; after being fully dissolved and membrane filtration, the sample was injected into the IC, and a chromatogram map of the sample was compared with a chromatogram map of the standard sample for quantitative analysis.

An OD235 judging basis: the main component of the seaweed raw material is alga oligosaccharide which is from algin. Under the action of algin lyase, C-O glucosidic bonds in guluronic acid and mannuronic acid in the algin are cut off to form oligosaccharide with unsaturated double-bond structure between non-reducing ends C4-5. The oligosaccharide with this structure has intensive absorption peak at 230-240 nm, and the content of the alga oligosaccharide can be represented by the adsorption intensity OD235 value at 235 nm.

An OD235 judging result of the first seaweed near water beverage: when the OD235 absorbance value>0.20, the content of the alga oligosaccharide in the sample was relatively high, and it was judged as grade I. When the OD235 absorbance value was between 0.15 and 0.20, under a shortage of raw material, it can be used for production by softening of terms, and the first seaweed near water beverage was judged as grade II. When the OD235 absorbance value was <0.15, the raw material was returned to the raw material plant directly and was not used for production.

### Example 4 Evaluation of an enhanced test for the seaweed near water beverage

The first seaweed near water beverage (the 3% seaweed stock solution was diluted to 0.01% seaweed enzyme powder solution) was taken for an enhanced predictive test, i.e., under sunlight exposure for 4 hours, the enhanced seaweed near water beverage was obtained, and was subjected to evaluation and index analysis. During enhancing, the exposure time was usually not less than 3.5 hours, preferably 4 hours; and after the first seaweed near water beverage was enhanced, floccules might increase, the turbidity may increase, and the color may become deeper.

### 4.1. Sensory and appearance Evaluation

The enhanced seaweed near water beverage was subjected to appearance detection; its color was measured by the EBC colorimeter; it was observed whether there were floccules or visible precipitates; and it was tasted to determine whether there was fishy smell. Corresponding grades of its color and luster and fishy smell are shown in Table 7.

Wherein, for the color evaluation, inject the enhanced seaweed near water beverage into the cuvette of the EBC colorimeter, and compare with the standard EBC color disc to read the chromaticity of the solution. When the chromaticity was <2 EBC and the color was colorless and transparent, it was marked as grade I; when the chromaticity was <2 EBC and the color was colorless with slight floccules, it was marked as grade II; and when 2EBC≤chromaticity < 15EBC and the color was light yellow with slight floccules, it was marked as grade III.

For the sensory evaluation, the fifth reference and the sixth reference in example 3 were adopted, and the enhanced seaweed near water sample was respectively compared with the two references to determine its grade. When the smell was close to that of the fifth reference, it was judged as free of fishy smell, and marked as grade I; when the smell was between that of the fifth reference and that of the sixth reference, it was judged as slight fishy smell, and marked as grade II; and when the smell was stronger than that of the sixth reference, it was judged as strong fishy smell, and marked as grade III.

**Table 7**

| Appearance | Colorless, transparent | Colorless with slight floccules | Light yellow with slight floccules |
|---|---|---|---|
| Chromaticity, EBC | <2 | <2 | ≥2 |
| Grade judgment | Grade I | Grade II | Grade III |
| Sense | Free of fishy smell | Slight fishy smell | Strong fishy smell |
| Grade judgment | Grade I | Grade II | Grade III |

### 4.2 Turbidity analysis

The turbidity of the enhanced seaweed near water beverage was analyzed by using the turbidity analyzer, and its turbidity and corresponding range are shown in Table 8.

**Table 8**

| Turbidity of enhanced seaweed product | <0.2 | 0.2-0.5 | >0.5 |
|---|---|---|---|
| Grade judgment | Grade I | Grade II | Grade III |

### 4.3 Evaluation of functional ingredients

An OD235 analysis method: the absorbance value of the enhanced seaweed near water beverage was measured at 235 nm by using a spectrophotometer, with water as a reference.

An OD235 judging result of the enhanced seaweed near water beverage: when the OD235 absorbance value>0.20, the content of the alga oligosaccharide in the sample was relatively high, and it was judged as grade I. When the OD235 absorbance value was between 0.15 and 0.20, and under a shortage of raw material, it can be used production by softening of terms, and the enhanced seaweed near water beverage was judged as grade II**.** When the OD235 absorbance value was <0.15, the raw material was returned to the raw material plant directly and was not used for production.

An oligosaccharide content analysis method: oligosaccharide in the seaweed raw material was analyzed by the ion chromatography (IC). Mannuronic acid monosaccharide, guluronic acid monosaccharide, and glucuronic acid monosaccharide were prepared into 100 ppm concentration, disaccharide to hexaose of mannuronic acid were prepared into 100 ppm, they were injected for analysis to establish a quantitative chromatogram; the seaweed enzyme powder was prepared into a 0.01% solution; after being fully dissolved and membrane filtration, the sample was injected into the IC, and a chromatogram of the sample was compared with the chromatogram of the standard sample for quantitative analysis.

A judging basis: the main component of the seaweed enzyme powder is uronic acid and polymers with different polymerization degrees. For analysis of the oligosaccharide, the ion chromatography was used to simultaneously analyze the monosaccharide composition and polymerization degree of the oligosaccharide in the seaweed enzyme powder. The saccharide components to be measured in the sample entered an ion exchange column system along with a KOH eluent and were separated according to the affinity difference of the separation column on anions ionized from the saccharides. The separated components were detected by an electrochemical detector. By using a single-point calibration method, the measured components were qualitated with retention time and quantified with peak area.

An oligosaccharide judging result of the enhanced seaweed near water beverage: when the content of the oligosaccharide was >45ppm, the content of the alga oligosaccharide in the sample was relatively high, and it was judged as grade I. When the content of the alga oligosaccharide was between 25 ppm and 45 ppm, under a shortage of raw material, it can be used for production by softening of terms, and the enhanced seaweed near water beverage was judged as grade II. When the content of the alga oligosaccharide was <25ppm, the raw material was returned to the raw material plant directly and was not used for production.

### Example 5 Establishment of raw material comprehensive evaluation indexes

Based on the analytical indexes in above examples 1-4, raw material comprehensive evaluation indexes were established, as shown in Table 9.

**Table 9**

| Index | Sample | Grade I | Grade II | Grade III |
|---|---|---|---|---|
| Appearance and color | Seaweed enzyme powder | Light yellow to light brown | Brown | Dark brown |
| | 3% seaweed stock solution | Light yellow | Brown | Dark brown |
| | Chromaticity of 3% seaweed stock solution, EBC | <15 | 15-25 | >25 |
| | First seaweed near water beverage | Colorless | Colorless | Light yellow |
| | Chromaticity of first seaweed near water beverage, EBC | <2 | <2 | ≥2 |
| | Enhanced seaweed near water beverage | Colorless | Colorless | Light yellow |
| | Chromaticity of enhanced seaweed near water beverage, EBC | <2 | <2 | ≥2 |
| Clarity | Particle size (Dx90) of seaweed enzyme powder, µm | <80 | 80-120 | >120 |
| | Molecular weight<10 thousand proportion % of 3% seaweed stock solution | >80 | 50-80 | <50 |
| | Turbidity of first seaweed near water beverage | <0.2 | 0.2-0.5 | >0.5 |
| | Turbidity of enhanced seaweed near water beverage | <0.2 | 0.2-0.5 | >0.5 |
| Sense | Seaweed enzyme powder | Mild fishy smell | Moderate fishy smell | Strong fishy smell |
| | Sense of 3% seaweed stock solution | Slight fishy smell | Mild fishy smell | Strong fishy smell |
| | Sense of first seaweed near water beverage | Free of fishy smell | Slight fishy smell | Strong fishy smell |
| | Sense of enhanced seaweed near water beverage | Free of fishy smell | Slight fishy smell | Strong fishy smell |
| Functional ingredients | OD235 of first seaweed near water beverage | >0.20 | 0.15-0.20 | 0.15-0.20 |
| | OD235 of enhanced seaweed near water beverage | >0.20 | 0.15-0.20 | 0.15-0.20 |
| | Oligosaccharide of first seaweed near water beverage, ppm | >45 | 25-45 | 25-45 |
| | Oligosaccharide of enhanced seaweed near water beverage, ppm | >45 | 25-45 | 25-45 |
| Conclusion | | Excellent quality | Quality with slightly defect | Poor quality |

### Example 6 Actual test of seaweed enzyme powder

Five kinds of seaweed enzyme powder were selected randomly in the present example, the indexes were analyzed in combination with the above examples 1-4, and the kinds of seaweed enzyme powder were graded according to index analysis. Specific data is shown in Table 10.

**Table 10**

| Index | Sample | Grade I | Grade I | Grade II | Grade III | Grade III |
|---|---|---|---|---|---|---|
| Appearance and color | Seaweed enzyme powder | Light yellow | Light yellow | Brown | Dark brown | Dark brown |
| | 3% seaweed stock solution | Light yellow | Light yellow | Brown | Dark brown | Dark brown |
| | Chromaticity of 3% seaweed stock solution, EBC | 9.1 | 13.4 | 21.1 | 26.2 | 36.2 |
| | First seaweed near water beverage | Colorless | Colorless | Colorless | Light yellow | Light yellow |
| | Chromaticity of first seaweed near water beverage, EBC | <2 | <2 | <2 | <2 | <2 |
| | Enhanced seaweed near water beverage | Colorless | Colorless | Colorless | Light yellow | Light yellow |
| | Chromaticity of enhanced seaweed near water beverage, EBC | <2 | <2 | <2 | <2 | <2 |
| Clarity | Particle size (Dx90) of seaweed enzyme powder, µm | 38 | 44 | 77 | 92 | 104 |
| | Molecular weight<10 thousand proportion % of 3% seaweed stock solution | 85 | 81 | 72 | 33 | 42 |
| | Turbidity of first seaweed near water beverage | 0.05 | 0.10 | 0.25 | 0.52 | 0.63 |
| | Turbidity of enhanced seaweed near water beverage | 0.08 | 0.12 | 0.32 | 0.58 | 0.67 |
| Sense | Seaweed enzyme powder | Mild fishy smell | Mild fishy smell | Moderate fishy smell | Strong fishy smell | Strong fishy smell |
| | Sense of 3% seaweed stock solution | Slight fishy smell | Slight fishy smell | Mild fishy smell | Strong fishy smell | Strong fishy smell |
| | Sense of first seaweed near water beverage | Free of fishy smell | Free of fishy smell | Slight fishy smell | Strong fishy smell | Strong fishy smell |
| | Sense of enhanced seaweed near water beverage | Free of fishy smell | Free of fishy smell | Slight fishy smell | Strong fishy smell | Strong fishy smell |
| Functional ingredients | OD235 of first seaweed near water beverage | 0.25 | 0.22 | 0.23 | 0.21 | 0.17 |
| | OD235 of enhanced seaweed near water beverage | 0.24 | 0.22 | 0.23 | 0.21 | 0.16 |
| | Oligosaccharide of first seaweed near water beverage, ppm | 48.7 | 52.5 | 35.1 | 26.2 | 28.9 |
| | Oligosaccharide of enhanced seaweed near water beverage, ppm | 48.4 | 51.8 | 34.6 | 25.3 | 28.3 |
| Conclusion | | Excellent quality | Excellent quality | Good quality | Poor quality | Poor quality |

### Example 7 Evaluation of actual beverage

In the present example, a mass production test was conducted on first seaweed near water beverages (the amount of seaweed enzyme powder was 100 ppm) adopting the five kinds of seaweed enzyme powder in example 6. Specifically, each seaweed enzyme powder was dissolved in an addition tank, with the concentration about 3% until it was fully and uniformly dissolved; the solution was centrifuged by a centrifuge and added into a mixing tank, and the volume was set; after being stabilized for 24 hours, the solution was subjected to processes such as filling, packaging, and sterilizing to prepare the first seaweed near water beverage. Index test data of the first seaweed near water beverages prepared based on the five kinds of seaweed enzyme powder are shown in Table 11.

**Table 11**

| Sample | Index | 1# | 2# | 3# | 4# | 5# |
|---|---|---|---|---|---|---|
| Seaweed enzyme powder | Quality grade | Grade I | Grade I | Grade II | Grade III | Grade III |
| | Appearance and color | Light yellow | Light yellow | Brown | Dark brown | Dark brown |
| | Sense | Mild fishy smell | Mild fishy smell | Moderate fishy smell | Strong fishy smell | Strong fishy smell |
| | Particle size (Dx90) of seaweed enzyme powder, µm | 38 | 44 | 77 | 92 | 104 |
| | Molecular weight<10 thousand proportion % of seaweed stock solution | 85 | 81 | 72 | 33 | 42 |
| First seaweed near water beverag e | Color of beverage | Colorless | Colorless | Colorless | Light yellow | Light yellow |
| | Sense of beverage | Free of fishy smell | Free of fishy smell | Slight fishy smell | Strong fishy smell | Strong fishy smell |
| | Turbidity of beverage | 0.05 | 0.10 | 0.25 | 0.52 | 0.63 |
| | OD235 of beverage | 0.24 | 0.22 | 0.23 | 0.21 | 0.16 |
| | Oligosaccharide , ppm | 49.5 | 54.5 | 37.1 | 25.8 | 28.3 |
| End of shelf life of first seaweed near water beverag e | Color of beverage | Colorless | Colorless | Colorless | Slightly yellow | Slightly yellow |
| | Sense of beverage | Free of fishy smell | Free of fishy smell | Slight fishy smell | Strong fishy smell | Strong fishy smell |
| | Turbidity of beverage | 0.08 | 0.13 | 0.30 | 0.55 | 0.66 |
| | OD235 of beverage | 0.23 | 0.22 | 0.22 | 0.20 | 0.16 |
| | Oligosaccharide , ppm | 50.3 | 52.5 | 35.1 | 25.2 | 28.6 |
| Quality of seaweed near water beverage | | Excellent quality | Excellent quality | Good quality when leaving factory; turbidity slightly increased at the end of shelf life. | Poor quality, unacceptable. | Poor quality, unacceptable. |

In combination with the data in the above tables, It can be seen that the rapid screening and evaluation method for a raw material of an seaweed near water beverage provided in the present application can be applied to the comprehensive evaluation of the seaweed raw material of the near water beverage, and formulates a next production process according to the evaluation result, so as to ensure that the seaweed near water beverage is clear and transparent in appearance, is odor-free and has significant functions.

This method covers the evaluation of the raw material, evaluation of the initial beverage, and evaluation of the entire shelf life of the beverage, and the evaluation phases are more comprehensive, thereby not only providing a technical base for ensuring the product quality comprehensively, but also acquiring the overall performance in the shelf life of the product based on evaluation of the raw material, thereby, greatly saving the economic cost.

## Claims

1. A rapid screening and evaluation method for a raw material seaweed enzyme powder used in a seaweed near water beverage, **characterised by** including the following steps:
analyzing appearance, fishy smell, and particle size of seaweed enzyme powder as the raw material;
analyzing appearance, fishy smell, and the percentage of components having molecular weight less than 10 thousand of a 3% (w/v) aqueous seaweed stock solution obtained based on the seaweed enzyme powder;
and analyzing its appearance, fishy smell, turbidity, and functional ingredients of a first seaweed near water beverage obtained based on the 3% (w/v) aqueous seaweed stock solution;
analyzing appearance, fishy smell, turbidity, and functional ingredients of an enhanced seaweed near water beverage; and
combining comprehensive results obtained from the above analysis, enabling the raw material of the seaweed near water beverage to be rapidly screened and evaluated.

2. The screening and evaluation method according to claim 1, wherein, the step of analyzing the appearance, the sense, and the percentage with molecular weight less than 10 thousand of the 3% seaweed stock solution obtained based on the seaweed enzyme powder includes:
color: when the color is light yellow, marked as grade I; when the color is brown, marked as grade II; and when the color is dark brown, marked as grade III;
the sense: when the smell is slight fishy smell, marked as grade I; when the smell is mild fishy smell, marked as grade II; and when the smell is strong fishy smell, marked as grade III; and,
the percentage with molecular weight less than 10 thousand: when percentage<50%, the solubility is poor; when 50%≤percentage≤80%, the solubility is average; and when percentage>80%, the solubility is good.

3. The screening and evaluation method according to claim 1, wherein, the step of analyzing the appearance, the sense, the turbidity, and the functional ingredients of the first seaweed near water beverage obtained based on the 3% seaweed stock solution includes:
color: when the color is colorless, marked as grade I; and when the color is light yellow, marked as grade II;
the sense: when the smell is free of fishy smell, marked as grade I; when the smell is slight fishy smell, marked as grade II; and when the smell is strong fishy smell, marked as grade III; and
the turbidity: when turbidity<0.2, marked as grade I; when 0.2≤turbidity≤0.5, marked as grade II; and when turbidity>0.5, marked as grade III.

4. The screening and evaluation method according to claim 3, wherein, the functional ingredients include a measured light absorption value OD235 at 235nm of the first seaweed near water beverage and a content of oligosaccharide contained in the first seaweed near water beverage.

5. The screening and evaluation method according to claim 4, wherein, the functional ingredients specifically are:
when light absorption value OD235>0.20, marked as grade I; when 0.15≤light absorption value OD235≤0.20, marked as grade II; and when light absorption value OD235<0.15, returning the raw material to a raw material plant directly; and,
when content of oligosaccharide>45ppm, marked as grade I; when 25ppm≤content of oligosaccharide≤45ppm, marked as grade II; and when content of oligosaccharide<25ppm, returning the raw material to the raw material plant directly.

6. The screening and evaluation method according to any one of claims 1-5, wherein, the enhanced seaweed near water beverage is a beverage obtained by exposing the first seaweed near water beverage to sunlight for 4 hours; wherein, the first seaweed near water beverage is a 0.01% seaweed enzyme powder solution obtained by diluting the 3% seaweed stock solution.

7. The screening and evaluation method according to claim 6, wherein, the step of analyzing the appearance, the sense, the turbidity, and the functional ingredients of the enhanced seaweed near water beverage includes:
color: when the color is colorless and transparent, marked as grade I; when the color is colorless with slight floccules, marked as grade II; and when the color is light yellow with slight floccules, marked as grade III;
the sense: when the smell is free of fishy smell, marked as grade I; when the smell is slight fishy smell, marked as grade II; and when the smell is strong fishy smell, marked as grade III; and
the turbidity: when turbidity<0.2, marked as grade I; when 0.2≤turbidity≤0.5, marked as grade II; and when turbidity>0.5, marked as grade III.

8. The screening and evaluation method according to claim 7, wherein, the functional ingredients include a measured light absorption value OD235 at 235nm of the enhanced seaweed near water beverage and a content of oligosaccharide contained in the enhanced seaweed near water beverage.

9. The screening and evaluation method according to claim 8, wherein, the functional ingredients specifically are:
when light absorption value OD235>0.20, marked as grade I; when 0.15≤light absorption value OD235≤0.20, marked as grade II; and when light absorption value OD235<0.15, returning the raw material to the raw material plant directly; and,
when content of oligosaccharide>45ppm, marked as grade I; when 25ppm≤content of oligosaccharide≤45ppm, marked as grade II; and when content of oligosaccharide<25ppm, returning the raw material to the raw material plant directly.

10. The screening and evaluation method according to any one of claims 1-5, wherein, the step of analyzing the appearance, the sense, and the particle size of the seaweed enzyme powder as raw material includes:
the appearance: when the color is light yellow to light brown, marked as grade I; when the color is brown, marked as grade II; and when the color is dark brown, marked as grade III;
the sense: when the smell is slight fishy smell to mild fishy smell, marked as grade I; when the smell is moderate fishy smell, marked as grade II; and when the smell is strong fishy smell, marked as grade III; and
the particle size: when particle size≤50µm, the solubility is excellent; when 50µm<particle size<80µm, the solubility is good; when 80µm≤particle size<120µm, the solubility is average; and when particle size≥120µm, the solubility is poor.

## Patentansprüche

1. Verfahren zum schnellen Screening und zur Bewertung von Algenenzympulver als Rohmaterial, das in einem Algen-Near-Water-Getränk verwendet wird, **dadurch gekennzeichnet, dass** es die folgenden Schritte umfasst:
Analyse des Aussehens, des fischartigen Geruchs und der Partikelgröße des Algenenzympulvers als Rohmaterial;
Analyse des Aussehens, des fischartigen Geruchs und des Prozentsatzes an Bestandteilen mit einem Molekulargewicht von weniger als 10.000 einer 3%igen (Gew./Vol.) wässrigen Algen-Stammlösung, die auf der Grundlage des Algenenzympulvers erhalten wird;
und Analyse des Aussehens, des fischartigen Geruchs, der Trübung und der funktionellen Inhaltsstoffe eines ersten Algen-Near-Water-Getränks, das auf der Grundlage der 3%igen (Gew./Vol.) wässrigen Algen-Stammlösung erhalten wird;
Analyse des Aussehens, des fischartigen Geruchs, der Trübung und der funktionellen Inhaltsstoffe eines angereicherten Algen-Near-Water-Getränks; und
Zusammenführung der aus den obigen Analysen gewonnenen umfassenden Ergebnisse, wodurch das Rohmaterial des Algen-Near-Water-Getränks schnell gescreent und bewertet werden kann.

2. Screening- und Bewertungsverfahren nach Anspruch 1, wobei der Schritt der Analyse des Aussehens, des Geruchs und des Prozentsatzes mit einem Molekulargewicht von weniger als 10.000 der 3%igen Algen-Stammlösung, die auf der Grundlage des Algenenzympulvers erhalten wird, Folgendes umfasst:
Farbe: wenn die Farbe hellgelb ist, wird dies als Klasse I bewertet; wenn die Farbe braun ist, wird dies als Klasse II bewertet; und wenn die Farbe dunkelbraun ist, wird dies als Klasse III bewertet;
Geruch: wenn der Geruch geringfügig fischartig ist, wird dies als Klasse I bewertet; wenn der Geruch leicht fischartig ist, wird dies als Klasse II bewertet; und wenn der Geruch stark fischartig ist, wird dies als Klasse III bewertet; und
Prozentsatz mit einem Molekulargewicht von weniger als 10.000: wenn der Prozentsatz < 50 % beträgt, ist die Löslichkeit schlecht; wenn 50 % ≤ Prozentsatz ≤ 80 %, ist die Löslichkeit durchschnittlich; und wenn der Prozentsatz > 80 %, beträgt, ist die Löslichkeit gut.

3. Screening- und Bewertungsverfahren nach Anspruch 1, wobei der Schritt der Analyse des Aussehens, des Geruchs, der Trübung und der funktionellen Inhaltsstoffe des ersten Algen-Near-Water-Getränks, das auf der Grundlage der 3%igen Algen-Stammlösung erhalten wird, Folgendes umfasst:
Farbe: wenn die Farbe farblos ist, wird dies als Klasse I bewertet; und wenn die Farbe hellgelb ist, wird dies als Klasse II bewertet;
Geruch: wenn der Geruch frei von fischartigem Geruch ist, wird dies als Klasse I bewertet; wenn der Geruch geringfügig fischartig ist, wird dies als Klasse II bewertet; und wenn der Geruch stark fischartig ist, wird dies als Klasse III bewertet; und
Trübung: wenn die Trübung < 0,2 ist, wird dies als Klasse I bewertet; wenn 0,2 ≤ Trübung ≤ 0,5, wird dies als Klasse II bewertet; und wenn die Trübung > 0,5 ist, wird dies als Klasse III bewertet.

4. Screening- und Bewertungsverfahren nach Anspruch 3, wobei die funktionellen Inhaltsstoffe einen gemessenen Lichtabsorptionswert OD235 bei 235 nm des ersten Algen-Near-Water-Getränks und einen Gehalt an Oligosacchariden umfassen, die in dem ersten Algen-Near-Water-Getränk enthalten sind.

5. Screening- und Bewertungsverfahren nach Anspruch 4, wobei die funktionellen Inhaltsstoffe konkret wie folgt sind:
wenn der Lichtabsorptionswert OD235 > 0,20 ist, wird dies als Klasse I bewertet; wenn 0,15 ≤ Lichtabsorptionswert OD235 ≤ 0,20, wird dies als Klasse II bewertet; und wenn der Lichtabsorptionswert OD235 < 0,15 ist, wird das Rohmaterial direkt an eine Rohmaterialanlage zurückgeschickt; und,
wenn der Gehalt an Oligosacchariden > 45 ppm beträgt, wird dies als Klasse I bewertet; wenn 25 ppm ≤ Gehalt an Oligosacchariden ≤ 45 ppm, wird dies als Klasse II bewertet; und wenn der Gehalt an Oligosacchariden < 25 ppm beträgt, wird das Rohmaterial direkt an die Rohmaterialanlage zurückgeschickt.

6. Screening- und Bewertungsverfahren nach einem der Ansprüche 1 bis 5, wobei das angereicherte Algen-Near-Water-Getränk ein Getränk ist, das durch 4-stündige Sonneneinstrahlung auf das erste Algen-Near-Water-Getränk erhalten wird; wobei das erste Algen-Near-Water-Getränk eine 0,01%ige Algenenzympulverlösung ist, die durch Verdünnen der 3%igen Algen-Stammlösung erhalten wird.

7. Screening- und Bewertungsverfahren nach Anspruch 6, wobei der Schritt der Analyse des Aussehens, des Geruchs, der Trübung und der funktionellen Inhaltsstoffe des angereicherten Algen-Near-Water-Getränks Folgendes umfasst:
Farbe: wenn die Farbe farblos und transparent ist, wird dies als Klasse I bewertet; wenn die Farbe farblos mit leichten Flocken ist, wird dies als Klasse II bewertet; und wenn die Farbe hellgelb mit leichten Flocken ist, wird dies als Klasse III bewertet;
Geruch: wenn der Geruch frei von fischartigem Geruch ist, wird dies als Klasse I bewertet; wenn der Geruch geringfügig fischartig ist, wird dies als Klasse II bewertet; und wenn der Geruch stark fischartig ist, wird dies als Klasse III bewertet; und
Trübung: wenn die Trübung < 0,2 ist, wird dies als Klasse I bewertet; wenn 0,2 ≤ Trübung ≤ 0,5, wird dies als Klasse II bewertet; und wenn die Trübung > 0,5 ist, wird dies als Klasse III bewertet.

8. Screening- und Bewertungsverfahren nach Anspruch 7, wobei die funktionellen Inhaltsstoffe einen gemessenen Lichtabsorptionswert OD235 bei 235 nm des angereicherten Algen-Near-Water-Getränks und einen Gehalt an Oligosacchariden umfassen, die in dem angereicherten Algen-Near-Water-Getränk enthalten sind.

9. Screening- und Bewertungsverfahren nach Anspruch 8, wobei die funktionellen Inhaltsstoffe konkret wie folgt sind:
wenn der Lichtabsorptionswert OD235 > 0,20 beträgt, wird dies als Klasse I bewertet; wenn 0,15 ≤ Lichtabsorptionswert OD235 ≤ 0,20, wird dies als Klasse II bewertet; und wenn der Lichtabsorptionswert OD235 < 0,15 beträgt, wird das Rohmaterial direkt an die Rohmaterialanlage zurückgeschickt; und
wenn der Gehalt an Oligosacchariden > 45 ppm beträgt, wird dies als Klasse I bewertet; wenn 25 ppm ≤ Gehalt an Oligosacchariden ≤ 45 ppm, wird dies als Klasse II bewertet; und wenn der Gehalt an Oligosacchariden < 25 ppm beträgt, wird das Rohmaterial direkt an die Rohmaterialanlage zurückgeschickt.

10. Screening- und Bewertungsverfahren nach einem der Ansprüche 1 bis 5, wobei der Schritt der Analyse des Aussehens, des Geruchs und der Partikelgröße des Algenenzympulvers als Rohmaterial Folgendes umfasst:
Aussehen: wenn die Farbe hellgelb bis hellbraun ist, wird dies als Klasse I bewertet; wenn die Farbe braun ist, wird dies als Klasse II bewertet; und wenn die Farbe dunkelbraun ist, wird dies als Klasse III bewertet;
Geruch: wenn der Geruch geringfügig fischartig bis leicht fischartig ist, wird dies als Klasse I bewertet; wenn der Geruch mäßig fischartig ist, wird dies als Klasse II bewertet; und wenn der Geruch stark fischartig ist, wird dies als Klasse III bewertet; und
Partikelgröße: wenn die Partikelgröße ≤ 50 µm beträgt, ist die Löslichkeit ausgezeichnet; wenn 50 µm < Partikelgröße < 80 µm, ist die Löslichkeit gut; wenn 80 µm ≤ Partikelgröße < 120 µm, ist die Löslichkeit durchschnittlich; und wenn die Partikelgröße ≥ 120 µm beträgt, ist die Löslichkeit schlecht.

## Revendications

1. Procédé de criblage et d'évaluation rapides d'une poudre enzymatique d'algues servant de matière première dans une boisson fonctionnelle à base d'algues, **caractérisé en ce qu'**il comprend les étapes suivantes :
l'analyse de l'aspect, de l'odeur de poisson et de la granulométrie de la poudre enzymatique d'algues servant de matière première ;
l'analyse de l'aspect, de l'odeur de poisson et du pourcentage des composants ayant un poids moléculaire inférieur à 10 000 d'une solution-mère aqueuse d'algues à 3 % (p/v) obtenue à partir de la poudre enzymatique d'algues ;
l'analyse de l'aspect, de l'odeur de poisson, de la turbidité et des ingrédients fonctionnels d'une première boisson fonctionnelle à base d'algues obtenue à partir de la solution-mère aqueuse d'algues à 3 % (p/v) ;
l'analyse de l'aspect, de l'odeur de poisson, de la turbidité et des ingrédients fonctionnels d'une boisson fonctionnelle à base d'algues enrichie ; et
la combinaison des résultats détaillés issus de l'analyse ci-dessus, permettant le criblage et l'évaluation rapides de la matière première de la boisson fonctionnelle à base d'algues.

2. Procédé de criblage et d'évaluation selon la revendication 1, dans lequel l'étape d'analyse de l'aspect, de l'odeur et du pourcentage des composants ayant un poids moléculaire inférieur à 10 000 de la solution-mère d'algues à 3 % obtenue à partir de la poudre enzymatique d'algues comprend :
la couleur : lorsque la couleur est jaune clair, elle est indiquée comme de grade I ; lorsque la couleur est brune, elle est indiquée comme de grade Il ; et lorsque la couleur est brun foncé, elle est indiquée comme de grade III ;
l'odeur : lorsque l'odeur est une légère odeur de poisson, elle est indiquée comme de grade I ; lorsque l'odeur est une odeur de poisson modérée, elle est indiquée comme de grade II ; et lorsque l'odeur est une forte odeur de poisson, elle est indiquée comme de grade III ; et
le pourcentage des composants ayant un poids moléculaire inférieur à 10 000 : lorsque le pourcentage est < 50 %, la solubilité est médiocre ; lorsque 50 % ≤ pourcentage ≤ 80 %, la solubilité est moyenne ; et lorsque le pourcentage > 80 %, la solubilité est bonne.

3. Procédé de criblage et d'évaluation selon la revendication 1, dans lequel l'étape d'analyse de l'aspect, de l'odeur, de la turbidité et des ingrédients fonctionnels de la première boisson fonctionnelle à base d'algues obtenue à partir de la solution-mère d'algues à 3 % comprend :
la couleur : lorsque la couleur est incolore, elle est indiquée comme de grade I ; et lorsque la couleur est jaune clair, elle est indiquée comme de grade II ;
l'odeur : lorsque l'odeur est dépourvue d'odeur de poisson, elle est indiquée comme de grade I ; lorsque l'odeur est une légère odeur de poisson, elle est indiquée comme de grade II ; et lorsque l'odeur est une forte odeur de poisson, elle est indiquée comme de grade III ; et
la turbidité : lorsque la turbidité < 0,2, elle est indiquée comme de grade I ; lorsque 0,2 ≤ turbidité ≤ 0,5, elle est indiquée comme de grade Il ; et lorsque la turbidité > 0,5, elle est indiquée comme de grade III.

4. Procédé de criblage et d'évaluation selon la revendication 3, dans lequel les ingrédients fonctionnels comprennent une valeur d'absorption lumineuse mesurée OD235 à 235 nm de la première boisson fonctionnelle à base d'algues et une teneur en oligosaccharides contenue dans la première boisson fonctionnelle à base d'algues.

5. Procédé de criblage et d'évaluation selon la revendication 4, dans lequel les ingrédients fonctionnels sont précisément :
lorsque la valeur d'absorption lumineuse OD235 > 0,20, indiqués comme de grade I ; lorsque 0,15 ≤ valeur d'absorption lumineuse OD235 ≤ 0,20, indiqués comme de grade II ; et lorsque la valeur d'absorption OD235 < 0,15, la matière première est renvoyée directement à l'usine de matières premières ; et
lorsque la teneur en oligosaccharides est > 45 ppm, indiqués comme de grade I ; lorsque 25 ppm ≤ teneur en oligosaccharides ≤ 45ppm, indiqués comme de grade Il ; et lorsque la teneur en oligosaccharides est < 25 ppm, la matière première est renvoyée directement à l'usine de matières premières.

6. Procédé de criblage et d'évaluation selon l'une quelconque des revendications 1 à 5, dans lequel la boisson fonctionnelle à base d'algues enrichie est une boisson obtenue en exposant la première boisson fonctionnelle à base d'algues à la lumière du soleil pendant 4 heures ; ladite première boisson fonctionnelle à base d'algues étant une solution à base de poudre enzymatique d'algues à 0,01 % obtenue par dilution de la solution-mère d'algues à 3 %.

7. Procédé de criblage et d'évaluation selon la revendication 6, dans lequel l'étape d'analyse de l'aspect, de l'odeur, de la turbidité et des ingrédients fonctionnels de la boisson fonctionnelle à base d'algues enrichie comprend :
la couleur : lorsque la couleur est incolore et transparente, elle est indiquée comme de grade I ; lorsque la couleur est incolore avec une légère floculation, elle est indiquée comme de grade II ; et lorsque la couleur est jaune clair avec une légère floculation, elle est indiquée comme de grade III ;
l'odeur : lorsque l'odeur est dépourvue d'odeur de poisson, elle est indiquée comme de grade I ; lorsque l'odeur est une légère odeur de poisson, elle est indiquée comme de grade Il ; et lorsque l'odeur est une forte odeur de poisson, elle est indiquée comme de grade III ; et
la turbidité : lorsque la turbidité est < 0,2, elle est indiquée comme de grade I ; lorsque 0,2 ≤ turbidité ≤ 0,5, elle est indiquée comme de grade Il ; et lorsque la turbidité est > 0,5, elle est indiquée comme de grade III.

8. Procédé de criblage et d'évaluation selon la revendication 7, dans lequel les ingrédients fonctionnels comprennent une valeur d'absorption lumineuse mesurée OD235 à 235 nm de la boisson fonctionnelle à base d'algues enrichie et une teneur en oligosaccharides contenus dans la boisson fonctionnelle à base d'algues enrichie.

9. Procédé de criblage et d'évaluation selon la revendication 8, dans lequel les ingrédients fonctionnels sont précisément :
lorsque la valeur d'absorption lumineuse OD235 > 0,20, indiqués comme de grade I ; lorsque 0,15 ≤ valeur d'absorption lumineuse OD235 ≤ 0,20, indiqués comme de grade II ; et lorsque la valeur d'absorption lumineuse OD235 < 0,15, la matière première est renvoyée directement à l'usine de matières premières ; et
lorsque la teneur en oligosaccharides est > 45 ppm, indiqués comme de grade I ; lorsque 25 ppm ≤ teneur en oligosaccharides ≤ 45 ppm, indiqués comme de grade Il ; et lorsque la teneur en oligosaccharides est < 25 ppm, la matière première est renvoyée directement à l'usine de matières premières.

10. Procédé de criblage et d'évaluation selon l'une quelconque des revendications 1 à 5, dans lequel l'étape d'analyse de l'aspect, de l'odeur et de la granulométrie de la poudre enzymatique d'algues servant de matière première comprend :
l'aspect : lorsque la couleur est jaune clair à brun clair, il est indiqué comme de grade I ; lorsque la couleur est brune, il est indiqué comme de grade Il ; et lorsque la couleur est brun foncé, il est indiqué comme de grade III ;
l'odeur : lorsque l'odeur est une légère odeur de poisson jusqu'à une odeur de poisson modérée, elle est indiquée comme de grade I ; lorsque l'odeur est une odeur de poisson moyenne, elle est indiquée comme de grade Il ; et lorsque l'odeur est une forte odeur de poisson, elle est indiquée comme de grade III ; et
la granulométrie : lorsque la granulométrie est ≤ 50 µm, la solubilité est excellente ; lorsque 50 µm ≤ granulométrie ≤ 80 µm, la solubilité est bonne ; lorsque 80 µm ≤ granulométrie ≤ 120 µm, la solubilité est moyenne ; et lorsque la granulométrie est ≥ 120 µm, la solubilité est médiocre.
